# EUROPEAN PATENT APPLICATION

(11) **EP 4 509 612 A1**
(43) Date of publication of application: **19.02.2025**
(21) Application number: 23787650.3
(22) Date of filing: 10.04.2023
(51) Int. Cl.: C12P 21/02, C12N 5/10, C12N 9/88, C12N 15/52, C12N 15/63

(54) **METHOD FOR PREPARING S-LACTOYLGLUTATHIONE**

(30) Priority: 12.04.2022 CN 202210381545
(71) Applicant: Mint Biotechnologies Co., Ltd., Hangzhou, Zhejiang 310012 (CN)
(72) Inventor: WANG, Yuchen, Hangzhou, Zhejiang 310012 (CN); ZHANG, Kechun, Hangzhou, Zhejiang 310012 (CN)
(74) Representative: Gille Hrabal Partnerschaftsgesellschaft mbB Patentanwälte
(86) International application number: PCT/CN2023/087385
(87) International publication number: WO 2023/198006

(57) **Abstract**

The present invention belongs to the technical field of genetic engineering and fermentation engineering, and in particular relates to a method for preparing S-lactoylglutathione, wherein glutamate, glycine, cysteine and methylglyoxal are used as raw materials, and are converted into S-lactoylglutathione under the catalysis of glutathione synthetase and glyoxalase. According to the present invention, the raw materials with relatively low cost are used for fermentation, the operation is simple, the conversion rate is high, the yield of the prepared S-lactoylglutathione is high, and the method is suitable for batch industrial production.

## Description

The present application claims priority to Chinese Patent Application No. 202210381545.6 filed to China National Intellectual Property Administration on Apr. 12, 2022, and entitled "METHOD FOR PREPARING S-LACTOYLGLUTATHIONE", which is incorporated herein by reference in its entirety.

### TECHNICAL FIELD

The present disclosure relates to the technical field of genetic engineering and fermentation engineering, and particularly relates to a preparation method for S-lactoylglutathione.

### BACKGROUND

Glutathione is a biologically active non-protein thiol compound widely found in organisms. Glutathione is a main antioxidant in the body, which can resist the destructive effect of oxidants on sulfhydryl groups, and protect proteins and enzymes containing sulfhydryl groups in cell membranes. The physiological functions of glutathione are mainly manifested in six aspects: (1) as the most abundant antioxidant in the cell, protecting DNA, proteins, and other biomolecules against oxidative damage; (2) performing detoxification of foreign substances through glutathione-S-transferase; (3) participating in the absorption and transport of amino acids; (4) participating in cell signaling and maintaining normal life activities of cells; (5) participating in the reduction of methemoglobin and promoting the absorption of iron; (6) regulating lymphocyte function, and resisting virus. Therefore, reduced glutathione is widely used in the fields of medicine, food, cosmetic, etc. However, due to the instability (easy to be oxidized) of the sulfhydryl group in the structure of glutathione, the storage, transportation, and application of glutathione are carried out under strict conditions, which restricts the further application of glutathione. Glutathione derivatives, which are relatively more stable and can be metabolized and decomposed back to glutathione, are therefore considered as an alternative to the supply of glutathione in the future. S-lactoylglutathione is produced from glutathione and methylglyoxal under the catalysis of glyoxalase, and is part of the detoxification pathway for methylglyoxal. In the detoxification pathway for methylglyoxal, firstly, S-lactoylglutathione is produced from methylglyoxal and glutathione in the presence of glyoxalase, and then hydrolyzed back to glutathione in the presence of S-lactoylglutathione hydrolase, in which lactic acid is also produced. Finally, the detoxification of methylglyoxal is completed through the lactic acid. The production of S-lactoylglutathione in the detoxification process described above can be regarded as utilizing the lactoyl group to protect the sulfhydryl group of glutathione which is easy to be oxidized, so as to obtain a more stable glutathione derivative which is not easy to be oxidized.

Currently, the research and development of S-lactoylglutathione synthesis are less, and the literature focuses on the direct synthesis of S-lactoylglutathione using glutathione and aldehydes under the *in vitro* enzymatic catalysis, which is a preparation method with a complicated enzyme extraction process and high cost of glutathione in the starting materials. Due to the high cost, it is difficult to achieve the industrial mass production of S-lactoylglutathione.

### SUMMARY

The technical problem to be solved by the present disclosure is to provide a preparation method for S-lactoylglutathione with low production cost and applicable for industrial production.

The second technical problem to be solved by the present disclosure is to improve the transformation rate in the production process and increase the yield of S-lactoylglutathione.

The present disclosure provides a preparation method for S-lactoylglutathione, wherein glutamic acid, glycine, cysteine, and methylglyoxal are used as starting materials and are converted into S-lactoylglutathione under the catalysis of glutathione synthetase and glyoxalase.

In some embodiments, the glutamic acid, glycine, cysteine, and methylglyoxal are used as substrates, a recombinant microorganism comprising a glutathione synthetase-encoding gene and a glyoxalase-encoding gene is added for fermentation, and the glutathione synthetase and the glyoxalase are produced by overexpression of the recombinant microorganism.

In some embodiments, the glutathione synthetase-encoding gene is selected from any one or more of gshF, gshA, and gshB, and is preferably gshF; further preferably, the nucleotide sequence of the gshF is set forth in SEQ ID NO: 1.

In some embodiments, the glyoxalase-encoding gene comprises gloA, wherein preferably, the nucleotide sequence of the gloA is set forth in SEQ ID NO: 2.

In some embodiments, the preparation method comprises constructing the recombinant microorganism by a genetic engineering method comprising plasmid expression or genomic integration.

In some embodiments, the recombinant microorganism is constructed by the plasmid expression method;
preferably, the construction method is as follows: a glutathione synthetase-encoding gene and a glyoxalase-encoding gene are obtained by PCR amplification, the obtained genes are co-ligated to a
plasmid vector comprising an IPTG inducible promoter and transformed into a competent cell, and after sequencing, a recombinant vector is obtained; and the recombinant vector is transformed into a recipient microorganism to obtain the recombinant microorganism;
preferably, the plasmid vector is selected from any one or two of pZAlac and pZElac.

In some embodiments, the recombinant vector is pZE-gshF-gloA;
preferably, a construction method for the pZE-gshF-gloA is as follows: the gshF gene and gloA gene are obtained by PCR amplification, co-ligated to the pZElac vector comprising an IPTG inducible promoter, and transformed into a competent cell, and after sequencing, the plasmid pZE-gshF-gloA is obtained;
preferably, the gshF gene and gloA gene are obtained by PCR amplification using the genome of *Escherichia coli* MG1655 as a template;
preferably, the competent cell is *Escherichia coli* dh5a.

In some embodiments, the recipient microorganism is selected from one or more of *Escherichia coli, Bacillus, Corynebacterium, Saccharomyces,* or *Streptomyces.*

In some embodiments, the recipient microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

In some embodiments, if the recipient microorganism comprises a gene expressing S-lactoylglutathione hydrolase, the gene expressing S-lactoylglutathione hydrolase on the recipient microorganism is required to be knocked out; the gene expressing S-lactoylglutathione hydrolase is, for example, gloB, gloC, or yeiG;
preferably, the nucleotide sequence of the gloB is set forth in SEQ ID NO: 5;
preferably, the nucleotide sequence of the gloC is set forth in SEQ ID NO: 6;
preferably, the nucleotide sequence of the yeiG is set forth in SEQ ID NO: 7;
preferably, the recipient microorganism is *Escherichia coli* MG1655ΔgloB, *Escherichia coli* MG1655ΔgloC, *Escherichia coli* MG1655ΔyeiG, *Escherichia coli* MG1655ΔgloBΔgloC, *Escherichia coli* MG1655ΔgloCΔyeiG, *Escherichia coli* MG1655ΔgloBΔyeiG, or *Escherichia coli* MG1655ΔgloBΔgloCΔyeiG.

In some embodiments, if the recipient microorganism comprises a gene expressing cysteine hydrolase or glutathione hydrolase, the gene expressing cysteine hydrolase or glutathione hydrolase on the recipient microorganism is further required to be knocked out;
preferably, the gene expressing cysteine hydrolase is tnaA; further preferably, the nucleotide sequence of the tnaA is set forth in SEQ ID NO: 3;
preferably, the gene expressing glutathione hydrolase is ggt; further preferably, the nucleotide sequence of the ggt is set forth in SEQ ID NO: 4;
preferably, the recipient microorganism is *Escherichia coli* MG1655ΔtnaA, *Escherichia coli* MG1655Δggt, or *Escherichia coli* MG1655ΔtnaAΔggt.

In some embodiments, the recipient microorganism is *Escherichia coli* MG1655ΔtnaAΔggtΔgloBΔgloCΔyeiG.

In some embodiments, a method for constructing the *Escherichia coli* MG1655ΔtnaAΔggtΔgloBΔgloCΔyeiG comprises the following steps:
1) knocking out the tnaA, ggt, gloB, gloC, and yeiG genes of a wild-type *Escherichia coli* MG1655 strain separately using a homologous recombination method to give five single-deletion bacteria strains;
2) adding the four single-deletion bacteria strains Δggt, ΔgloB, ΔgloC, and ΔyeiG obtained in Step 1) into a wild-type P1 phage separately and culturing to give phages P1vir ggt, P1vir gloB, P1vir gloC, and P1vir yeiG comprising *Escherichia coli* gene fragments with ggt, gloB, gloC, and yeiG knockout characters, respectively; and
3) transfecting the ΔtnaA single-deletion bacteria strain obtained in Step 1), as a recipient strain, by adding the phages obtained in Step 2) sequentially to give *Escherichia coli* MG1655ΔtnaAΔggtΔgloBΔgloCΔyeiG.

In some embodiments, during the fermentation, the fermentation temperature is 20-90 °C.

In some embodiments, the molar concentration ratio of the glutamic acid to the glycine to the cysteine to the methylglyoxal is 8-12:8-12:6-10:1-4.

In some embodiments, during the fermentation, the glutamic acid, glycine, cysteine, and a recombinant microorganism are first added, the fermentation culture is preferably performed for 1-4 h to accumulate glutathione, the methylglyoxal is then added, and the fermentation is continued; preferably, during the fermentation, the concentration of the methylglyoxal in a fermentor is maintained to be 0.2-4 mM by using a slow fed-batch addition method.

The present disclosure further provides a preparation method for S-lactoylglutathione, wherein glutathione and methylglyoxal are used as starting materials and are converted into S-lactoylglutathione under the catalysis of a recombinant microorganism overexpressing glyoxalase or its crude enzyme extracts thereof;
preferably, a gene expressing S-lactoylglutathione hydrolase in the recombinant microorganism is knocked out, and the gene expressing S-lactoylglutathione hydrolase is, for example, gloB, gloC, or yeiG;
preferably, the nucleotide sequence of the gloB is set forth in SEQ ID NO: 5;
preferably, the nucleotide sequence of the gloC is set forth in SEQ ID NO: 6;
preferably, the nucleotide sequence of the yeiG is set forth in SEQ ID NO: 7;
preferably, the recombinant microorganism is *Escherichia coli* MG1655ΔgloB, *Escherichia coli* MG1655ΔgloC, *Escherichia coli* MG1655ΔyeiG, *Escherichia coli* MG1655ΔgloBΔgloC, *Escherichia coli* MG1655ΔgloCΔyeiG, *Escherichia coli* MG1655ΔgloBΔyeiG, or *Escherichia coli* MG1655ΔgloBΔgloCΔyeiG.

The present disclosure further provides a recombinant microorganism for preparing S-lactoylglutathione, wherein the recombinant microorganism overexpresses an endogenous or exogenous glutathione synthetase-encoding gene and glyoxalase-encoding gene;
preferably, the glutathione synthetase-encoding gene is selected from any one or more of gshF, gshA, and gshB, and is preferably gshF; further preferably, the nucleotide sequence of the gshF is set forth in SEQ ID NO: 1;
preferably, the glyoxalase-encoding gene comprises gloA; preferably, the nucleotide sequence of the gloA is set forth in SEQ ID NO: 2;
preferably, the recipient microorganism is selected from one or more of *Escherichia coli, Bacillus, Corynebacterium, Saccharomyces,* or *Streptomyces;* Further preferably, the recipient microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

The present disclosure further provides a recombinant DNA or biomaterial for preparing S-lactoylglutathione, wherein the recombinant DNA or biomaterial comprises a glutathione synthetase-encoding gene and a glyoxalase-encoding gene;
preferably, the glutathione synthetase-encoding gene is selected from any one or more of gshF, gshA, and gshB, and is preferably gshF; further preferably, the nucleotide sequence of the gshF is set forth in SEQ ID NO: 1;
preferably, the glyoxalase-encoding gene comprises gloA; preferably, the nucleotide sequence of the gloA is set forth in SEQ ID NO: 2;
preferably, the biomaterial is an expression cassette, a transposon, a plasmid vector, a phage vector, or a virus vector.

The present disclosure further provides use of the recombinant microorganism, the recombinant DNA or biomaterial described above for manufacturing S-lactoylglutathione.

Beneficial effects: The preparation method for S-lactoylglutathione described herein is simple in preparation process and convenient to operate, with significantly reduced starting material cost, and high product transformation rate and yield as compared to methods directly using glutathione as a starting material, so it is applicable for industrial mass production.

### BRIEF DESCRIPTION OF THE DRAWINGS

FIG. 1 is a schematic diagram showing the results of S-lactoylglutathione production under different conditions in Example 1.
FIG. 2 is a schematic diagram of the gene knockout of the recipient strain.

### DETAILED DESCRIPTION

In order to make the technical means, creation characteristics, achieved purposes, and effects of the present disclosure easy to understand, the present disclosure is further illustrated below with reference to specific examples.

In the present disclosure, unless otherwise indicated, scientific and technical terms have the same meaning as commonly understood by those skilled in the art. In addition, the terms and laboratory procedures related to nucleic acid chemistry, molecular biology, cell and tissue culture, microbiology, and immunology used herein are the terms and conventional procedures widely used in the corresponding fields. Also, in order to better understand the present disclosure, the definitions and interpretations of the related terms are provided below.

It should be appreciated that the terms used herein are for the purpose of illustrating particular embodiments only, and are not intended to be limiting.

The articles "a", "an" and "the" are used herein to refer to one or more than one of the grammatical object of the article.

The use of alternatives (e.g., "or") should be understood to refer to one, two, or any combination of the alternatives. The term "and/or" should be interpreted as referring to one or both of the alternatives.

As used herein, the term "gene synthesis" refers to a generation process using recombinant DNA techniques or a production process using DNA or amino acid sequence synthesis techniques available and well known in the art.

The term "encode" or "code" refers to the inherent property of specific sequences of nucleotides in a polynucleotide, such as a gene, a cDNA, or an mRNA, to serve as a template in synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of an mRNA corresponding to the gene produces the protein in a cell or other biological system. Both the coding strand, which comprises a nucleotide sequence equivalent to the mRNA sequence and is usually provided in a sequence listing, and the non-coding strand, which is used as a template for transcription of a gene or cDNA, may be referred to as encoding the protein or other product of that gene or cDNA.

As used herein, the term "endogenous" refers to any substance derived from or produced within an organism, cell, tissue or system.

As used herein, the term "exogenous" refers to any substance introduced into or produced outside of an organism, cell, tissue or system.

As used herein, the term "expression" is defined as the transcription and/or translation of a particular nucleotide sequence driven by its promoter.

Unless otherwise specified, the "polynucleotide sequence encoding an amino acid sequence" includes all nucleotide sequences that are degenerate versions of each other and encode the same amino acid sequence. The phrase "nucleotide sequence encoding a protein or an RNA" may also include introns to the extent that the nucleotide sequence encoding the protein may contain an intron(s) in some versions.

As used herein, the term "vector" refers to a composition of matter that comprises an isolated nucleic acid and can be used to deliver the isolated nucleic acid to the interior of a cell. The transferred nucleic acid is typically ligated to, e.g., inserted into, a vector nucleic acid molecule. A vector may comprise sequences that direct autonomous replication in the cell, or may comprise sequences sufficient to allow integration into the host cell DNA. Many vectors are known in the art, including but not limited to plasmids, phagemids, artificial chromosomes, bacteriophages and animal viruses. Therefore, the term "vector" encompasses an autonomously replicating plasmid or virus.

The DNA polymerase Phanta Max Super-Fidelity DNA Polymerase and the non-ligase-dependent single-fragment quick cloning kit ClonExpress^{®}II One Step used in the embodiments of the present disclosure are purchased from Nanjing Vazyme Biotech Co., Ltd.

The recombinant vectors used in some examples are constructed as follows:
Primers were designed separately based on the genome of *Escherichia coli* MG1655 and the genome of *Streptococcus thermophilus* published by NCBI: The combining portion of the primer and the template was represented by capital letters with a Tm value of 58-62 °C, and the homologous arm portions were represented by English letters in lowercase and were all 20 bp:
gshF_F: ttaaagaggagaaaggtaccATGACATTAAACCAACTTCTTCAAAAACTGG
gshF_R: ttaatttctcctgtcgacTTAAGTTTGACCAGCCACTATTTCTGG
gloA_F: gtcgacaggagaaattaactATGCGTCTTCTTCATACCATGCTG
gloA_R: ttgatgcctctagaaagcttTTAGTTGCCCAGACCGCG
A gloA gene fragment was obtained by PCR amplification by using the genome of *Escherichia coli* MG1655 as a template, and a gshF gene fragment was obtained by PCR amplification by using the genome of *Streptococcus thermophilus* as a template. The genes were co-ligated to a vector pZElac comprising an IPTG inducible promoter by a non-ligase-dependent single-fragment quick cloning kit, and then transformed into BW25113 competent cells. A kanamycin sulfate resistance plate was coated with the cells for culturing overnight. Positive clones were selected for sequencing verification, and the correct recombinant vector was designated as pZE-gshF_gloA.

Among them:
The nucleotide sequence of the gshF is set forth in SEQ ID NO: 1.
The nucleotide sequence of the gloA is set forth in SEQ ID NO: 2.

The recipient strains used in some examples are constructed as follows:
Construction of *Escherichia coli* MG1655ΔtnaAΔggtΔgloBΔgloCΔyeiG strain:
1) The tnaA, ggt, gloB, gloC, and yeiG genes of a wild-type *Escherichia coli* MG1655 strain were separately knocked out by a homologous recombination method to give five single-deletion bacteria strains; the prepared five single-deletion bacteria strains all have anti-kanamycin sulfate resistance genes.
2) The four single-deletion bacteria strains Δggt, ΔgloB, ΔgloC, and ΔyeiG with anti-kanamycin sulfate resistance genes prepared by the homologous recombination method in Step 1) were cultured at 37 °C overnight, and then transferred into a LB culture medium containing 5 mmol/L CaCl₂ and 0.1% glucose. The mixture was cultured at 37 °C for 1 h, and then, a wild-type P1 phage was added. The mixture was cultured for another 1-3 h. A few drops of chloroform were added, and the mixture was further cultured for 3-8 min. The mixture was centrifuged, and the supernatant was collected to give phages P1vir ggt, P1vir gloB, P1vir gloC, and P1vir yeiG comprising *Escherichia coli* gene fragments with ggt, gloB, gloC, and yeiG knockout characters, respectively.
3) The ΔtnaA single-deletion bacteria strain obtained in Step 1), as a recipient strain, was transfected by adding the phages obtained in Step 2) sequentially to give *Escherichia coli* MG1655ΔtnaAΔggtΔgloBΔgloCΔyeiG. The specific steps of the method were as follows: The ΔtnaA single-deletion bacteria strain was used as a recipient strain to transformed a pCP20 plasmid and express a flippase recombinase gene. The homologous recombination of FRT sites was promoted, and anti-kanamycin sulfate resistance genes were knocked out. As a temperature-sensitive plasmid, pCP20 can be removed by changing environmental temperature. The ΔtnaA recipient strain was cultured overnight, and 1.5 mL bacterial solution was taken and centrifuged at 10000 g for 2 min. Then, the ΔtnaA recipient strain was resuspended with 0.75 mL P1 salt solution (10 mM CaCl₂ and 5 mM MgSO₄ in water), and 100 µL P1vir ggt phage was mixed with 100 µL ΔtnaA recipient strain suspension. The mixture was incubated at 37 °C for 30 min, and then 1 mL LB culture medium and 200 µL sodium citrate at 1 mol/L were added. The mixture was cultured at 37 °C for another 1 h, and then centrifuged to collect bacterial cells. After being resuspended with a 100 µL LB culture medium, a LB plate containing kanamycin (the concentration of kanamycin was 50 µg/mL) was coated with the bacterial cells, and cultured at 37 °C overnight. Then, clones were selected for PCR amplification identification, and the positive clone was ΔtnaAΔggt comprising the anti-kanamycin sulfate resistance gene. The ΔtnaAΔggt comprising anti-kanamycin sulfate resistance gene was used as a recipient strain, and after the anti-kanamycin sulfate resistance gene was knocked out with pcp20, was transfected with P1vir gloB to give ΔtnaAΔggtΔgloB. By analogy, the ΔtnaAΔggtΔgloBΔgloCΔyeiG target strain was obtained by repeated knockout of the anti-kanamycin sulfate resistance genes of the ΔtnaAΔggtΔgloBΔgloCΔyeiG strain obtained during the transfection process, and further knockout of the anti-kanamycin sulfate resistance gene with pcp20. During this process, the transfection order was not limited.

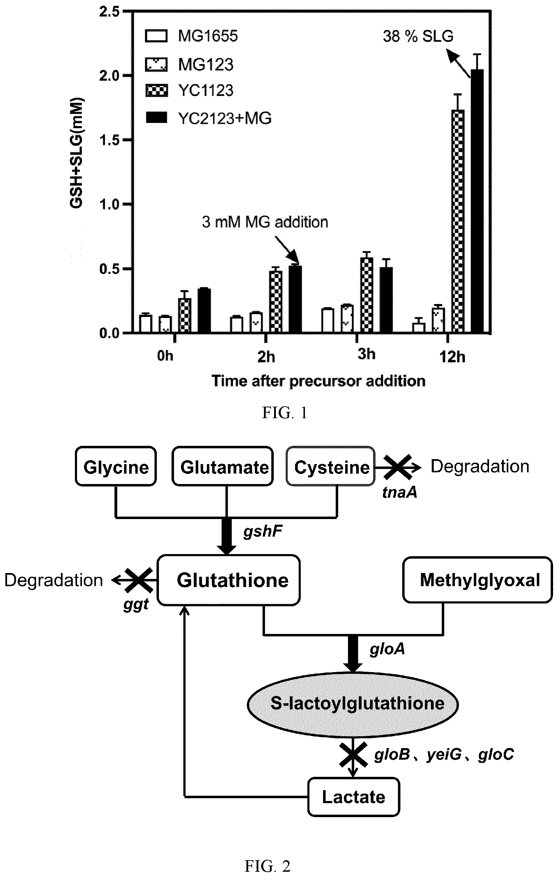

The gloB, gloC, and yeiG were all S-lactoylglutathione hydrolase, and the product yield can be further improved after the knockout of tnaA and ggt. The concept principle is shown in FIG. 2.

The nucleotide sequence of the tnaA is set forth in SEQ ID NO: 3.

The nucleotide sequence of the ggt is set forth in SEQ ID NO: 4.

The nucleotide sequence of the gloB is set forth in SEQ ID NO: 5.

The nucleotide sequence of the gloC is set forth in SEQ ID NO: 6.

The nucleotide sequence of the yeiG is set forth in SEQ ID NO: 7.

### Example 1

The example shows the synthesis of S-lactoylglutathione in a shake flask.

The recombinant plasmid pZE-gshF_gloA was electroporated into *Escherichia coli* MG1655ΔtnaAΔggtΔgloBΔgloCΔyeiG, and positive clones were screened by using an ampicillin resistance gene as a selection marker. The transformant was seeded into a 2 mL LB culture medium, and cultured for 12 h. The activated strain obtained was seeded into an M9 culture medium containing one-thousandth of ampicillin and 20 g/L glucose in the inoculation amount of 1%, and the mixture was cultured in a 150 mL Erlenmeyer flask (liquid volume 15 mL, containing 0.5 g CaCO₃) at 30 °C with a rotation speed of 240 rpm until the OD value reached 0.4-0.6. IPTG was added to a final concentration of 0.2 mM, and 10 mM glutamic acid, 10 mM glycine, and 8 mM cysteine (wherein a lower proportion of cysteine could reduce the toxicity to the *Escherichia coli,* and was conducive to reducing the cost without reducing the yield) were added in one portion. As methylglyoxal has certain toxicity to *Escherichia coli,* upon certain glutathione accumulation after continuous culture and fermentation at 37 °C for 2 h, methylglyoxal was added again to 3 mM, and the mixture was fermented continuously for another 12 h. The fermentation broth was then collected, and the concentration of glutathione and S-lactoylglutathione was 1.3 mM and 0.8 mM, respectively, as detected by high performance liquid chromatography, with a glutathione transformation rate of 38.1%. Transformation rate = S-lactoylglutathione/(S-lactoylglutathione final yield + glutathione final yield).

As a Reference Example 1, the fermentation was performed directly by using a wild strain of *Escherichia coli* MG1655, and the inoculation and fermentation were performed under the same conditions. The fermentation broth was collected, and the concentration of glutathione was 0.08 mM as detected by high performance liquid chromatography, while no S-lactoylglutathione was detected.

As a Reference Example 2, the fermentation was performed directly by using *Escherichia coli* MG1655ΔtnaAΔggtΔgloBΔgloCΔyeiG, and the inoculation and fermentation were performed under the same conditions. The fermentation broth was collected, and the concentration of glutathione was 0.20 mM as detected by high performance liquid chromatography, while no S-lactoylglutathione was detected.

As a Reference Example 3, after the recombinant vector comprising only the gshF gene was transformed into *Escherichia coli* MG1655ΔtnaAΔggtΔgloBΔgloCΔyeiG, the inoculation and fermentation were performed under the same conditions. The fermentation broth was collected, and the concentration of glutathione was 1.7 mM as detected by high performance liquid chromatography, while no S-lactoylglutathione was detected.

Data results are shown in FIG. 1.

In the figure, MG1655 represents Reference Example 1, MG123 represents Reference Example 2, YC1123 represents Reference Example 3, and YC2123+MG (methylglyoxal) represents Example 1. The total content of glutathione and S-lactoylglutathione were measured and shown in the vertical coordinate of the graph, and no SLG (S-lactoylglutathione) was detected in any of the groups except for the data at 12 h of the YC2123+MG (methylglyoxal) group annotated in the graph, which contained 38% SLG.

Therefore, the technical solutions of the present disclosure can effectively improve the output and yield of S-lactoylglutathione.

### Example 2

The example shows the synthesis of S-lactoylglutathione in a fermentor.

The recombinant plasmid pZE-gshF_gloA was electroporated into *Escherichia coli* MG1655ΔtnaAΔggtΔgloBΔgloCΔyeiG, and positive clones were screened by using an ampicillin resistance gene as a selection marker. The single colonies of the recombinant bacteria described above were separately seeded into a 50 mL LB liquid medium containing 100 µg/mL ampicillin, and the mixture was cultured at 37 °C and 220 rpm for 14 h. The strain was then seeded into an M9 culture medium containing ampicillin and 50 g/L glucose, and the mixture was cultured and fermented in a 1 L fermentor (liquid volume 500 mL) at a rotation speed of 600 rpm for 6 h. IPTG was added to a final concentration of 0.1 mM, and 25 mM glutamic acid, 25 mM glycine, and 17 mM cysteine were added in one portion. In the experiment, it was found that adding 3 mM methylglyoxal in one portion had an effect on the growth of *Escherichia coli,* and thus the concentration of methylglyoxal was controlled to be maintained at 1 mM by slow fed-batch addition during the fermentation. The mixture was fermented continuously for another 24 h, and the fermentation broth was collected. The concentration of glutathione and S-lactoylglutathione was 2.1 g/L and about 2.8 g/L, respectively, as detected by high performance liquid chromatography, with a glutathione transformation rate of 57.1%.

### Example 3

The example compares the knockout effect between the recipient strain crude enzyme extracts and the hydrolase in the catalytic action for synthesizing S-lactoylglutathione.

The pZE-gloA was electroporated into *Escherichia coli* MG1655ΔtnaAΔggt and MG1655ΔtnaAΔggtΔgloBΔgloCΔyeiG, and positive clones were screened by using an ampicillin resistance gene as a selection marker. The transformant was seeded into a 2 mL LB culture medium, and cultured for 12 h. The activated strain obtained was seeded into a 2XYT culture medium containing one-thousandth of ampicillin in an amount of 1%, and the mixture was cultured in a 150 mL Erlenmeyer flask (liquid volume 15 mL) at 30 °C with a rotation speed of 240 rpm until the OD value reached 0.4-0.6. IPTG was added to a final concentration of 0.2 mM. After culturing at 16 °C with a rotation speed of 240 rpm for 20 h, the mixture was centrifuged at 18000 g, and the supernatant was discarded. The mixture was washed with 10 mM potassium phosphate buffer (pH 7.0) twice, and centrifuged. Then, the precipitate was resuspended with a 5 mL potassium phosphate buffer at 10 mM (pH 7.0). The resuspension was subjected to ultrasonic treatment at 0 °C for 5 min, and centrifuged at 18000 g for 40 min. Then, the supernatant was taken as the crude enzyme extracts.

In a 1 mL reaction system with a molar ratio of methylglyoxal to glutathione of 1:1, a buffer and 200 µL crude enzyme extracts were added, and the mixture was allowed to react in a water bath at 37 °C. Taking 10 mM methylglyoxal and 10 mM glutathione as an example, samples were taken and assayed after 2 h. In the MG1655ΔtnaAΔggt and MG1655ΔtnaAΔggtΔgloBΔgloCΔyeiG groups, glutathione was consumed completely, and 9.79 mM S-lactoylglutathione and 9.91 mM S-lactoylglutathione were produced, respectively. After another 12 h, samples were taken and assayed. It showed that the concentration of S-lactoylglutathione was 8.52 mM and 9.67 mM, respectively, indicating that the knockout of hydrolase genes gloB, gloC, and yeiG reduced the hydrolysis of S-lactoylglutathione.

The foregoing shows and describes the general principles, principal features, and advantages of the present disclosure. It should be understood by those skilled in the art that the present disclosure is not limited to the examples described above, and the examples described above and the description in the specification are merely illustration of the principles of the present disclosure. Various changes and modifications may be made without departing from the spirit and scope of the present disclosure, and such changes and modifications are within the protection scope of the present disclosure as claimed. The protection scope of the present disclosure as claimed is defined by the appended claims and equivalents thereof.

Sequence information
Sequence NO: 1
Sequence length: 2265
Sequence type: DNA
Sequence source: Streptococcus thermophilus

atgacattaa accaacttct tcaaaaactg gaagctacca gccctattct ccaagctaat 60

tttggaatcg agcgcgagag tctacgtgtc gataggcaag gacaactggt gcatacacct 120

cacccatcct gtctaggagc tcgtagtttc cacccctata ttcagactga tttttgcgag 180

tttcagatgg aactcatcac accagttgcc aaatctacta ctgaggctcg ccgatttctg 240

ggagctatta ctgatgtagc aggccgctct attgctacag acgaggttct ctggcctttg 300

tccatgccac ctcgtctaaa ggcagaggag attcaagttg ctcaactgga aaatgacttc 360

gaacgccatt atcgtaacta tttggctgaa aaatacggaa ctaaactaca agctatctca 420

ggtatccact ataatatgga actgggtaaa gatttagttg aggccttgtt ccaagaaagt 480

gatcagaccg atatgattgc cttcaaaaac gccctctatc ttaagctggc tcagaactac 540

ttgcgctacc gttgggtgat tacctatctc tttggggcct cacccatcgc cgaacaaggt 600

ttctttgacc aggaagttcc agaacctgtg cgttccttcc gtaacagtga ccacggctat 660

gtcaataagg aagagattca agtatccttt gtaagtctag aagattatgt ctcagccatt 720

gaaacctata tcgaacaagg agatttgaat gcagagaaag aattttactc agctgttcgt 780

ttccgtggac aaaaggttaa tcgttccttc cttgacaaag gaatcaccta cctagagttc 840

cgtaatttcg accttaaccc ttttgagcgt atcggtatta gtcagactac tatggacact 900

gtgcacttac tcattttagc cttcctttgg cttgatagcc ctgaaaatgt cgaccaagct 960

cttgcacaag gccacgcgtt aaatgagaaa attgccctct ctcatcctct aaaacctcta 1020

ccttcggagg ctaaaactca ggacattgta actgccctag accaactggt gcaacacttt 1080

ggacttggtg actatcatca agatctggtt aaacaagtta aggcagcctt tgcggatcca 1140

aatcaaacgc tctctgccca gctcttaccc tatatcaaag acaaatctct agccgaattt 1200

gctttaaaca aggctcttgc ctatcatgat tacgactgga ctgcccacta tgctctcaag 1260

ggctatgaag agatggaact ctccacccag atgttgctct ttgatgccat ccaaaaggga 1320

attcactttg aaatattgga tgagcaagat caattcctaa aactttggca ccaagaccat 1380

gttgaatacg tcaaaaacgg taacatgacc tcaaaagaca actacgtggt tccccttgct 1440

atggctaata agaccgtaac caagaagatt ctagcagatg ctggcttttc agttccttca 1500

ggagacgaat ttaccagtct tgaggaagga cttgcctact accctcttat caaggataag 1560

caaattgttg tcaaacccaa gtcaactaac tttggtctgg gaatttccat tttccaagaa 1620

cctgccagtc ttgacaacta tcaaaaagcc cttgaaattg ctttcgcaga agatacctct 1680

gtccttgttg aagaatttat tccaggaacc gaataccgtt tcttcatctt ggatgggcgt 1740

tgtgaggctg tgcttctgcg tgtcgctgcc aatgttattg gtgatggcaa acacaccatt 1800

cgtgaactag tcgctcagaa aaatgctaat ccattgcgtg gccgtgatca ccggtcacct 1860

ctggaaatca ttgagctagg agacatcgaa caactaatgt tagctcaaca gggttataca 1920

cctgatgata ttctcccaga aggaaaaaag gtcaatctgc gtcgtaattc caacatctct 1980

acaggtggtg actctattga tgtcactgag accatggatt cctcttacca agaattagcc 2040

gcagccatgg caactagcat gggcgcctgg gcttgcgggg ttgatctgat aattccagat 2100

gaaactcaaa ttgccaccaa ggaaaatcct cattgcacct gcattgagct caactttaac 2160

ccttcgatgt atatgcacac ctactgtgct gagggtcctg gccaagctat cactactaaa 2220

atcctagata aactttttcc agaaatagtg gctggtcaaa cttaa 2265

Sequence NO: 2
Sequence length: 408
Sequence type: DNA
Sequence source: Escherichia coli

atgcgtcttc ttcataccat gctgcgcgtt ggcgatttgc aacgctccat cgatttttat 60

accaaagtgc tgggcatgaa actgctgcgt accagcgaaa acccggaata caaatactca 120

ctggcgtttg ttggctacgg cccggaaacc gaagaagcgg tgattgaact gacctacaac 180

tggggcgtgg ataaatacga actcggcact gcttatggtc acatcgcgct tagcgtagat 240

aacgccgctg aagcgtgcga aaaaatccgt caaaacgggg gtaacgtgac ccgtgaagcg 300

ggtccggtaa aaggcggtac tacggttatc gcgtttgtgg aagatccgga cggttacaaa 360

attgagttaa tcgaagagaa agacgccggt cgcggtctgg gcaactaa 408

Sequence NO: 3
Sequence length: 1417
Sequence type: DNA
Sequence source: Escherichia coli

<400> 3
aatggaaaac tttaaacatc tccctgaacc gttccgcatt cgtgttattg agccagtaaa 60
acgtaccact cgcgcttatc gtgaagaggc aattattaaa tccggtatga acccgttcct 120
gctggatagc gaagatgttt ttatcgattt actgaccgac agcggcaccg gggcggtgac 180
gcagagcatg caggctgcga tgatgcgcgg cgacgaagcc tacagcggca gtcgtagcta 240
ctatgcgtta gccgagtcag tgaaaaatat ctttggttat caatacacca ttccgactca 300
ccagggccgt ggcgcagagc aaatctatat tccggtactg attaaaaaac gcgagcagga 360
aaaaggcctg gatcgcagca aaatggtggc gttctctaac tatttctttg ataccacgca 420
gggccatagc cagatcaacg gctgtaccgt gcgtaacgtc tatatcaaag aagccttcga 480
tacgggcgtg cgttacgact ttaaaggcaa ctttgacctt gagggattag aacgcggtat 540
tgaagaagtt ggtccgaata acgtgccgta tatcgttgca accatcacca gtaactctgc 600
aggtggtcag ccggtttcac tggcaaactt aaaagcgatg tacagcatcg cgaagaaata 660
cgatattccg gtggtaatgg actccgcgcg ctttgctgaa aacgcctatt tcatcaagca 720
gcgtgaagca gaatacaaag actggaccat cgagcagatc acccgcgaaa cctacaaata 780
tgccgatatg ctggcgatgt ccgccaagaa agatgcgatg gtgccgatgg gcggcctgct 840
gtgcatgaaa gacgacagct tctttgatgt gtacaccgag tgcagaaccc tttgcgtggt 900
gcaggaaggc ttcccgacat atggcggcct ggaaggcggc gcgatggagc gtctggcggt 960
aggtctgtat gacggcatga atctcgactg gctggcttat cgtatcgcgc aggtacagta 1020
tctggtcgat ggtctggaag agattggcgt tgtctgccag caggcgggcg gtcacgcggc 1080
attcgttgat gccggtaaac tgttgccgca tatcccggca gaccagttcc cggcacaggc 1140
gctggcctgc gagctgtata aagtcgccgg tatccgtgcg gtagaaattg gctctttcct 1200
gttaggccgc gatccgaaaa ccggtaaaca actgccatgc ccggctgaac tgctgcgttt 1260
aaccattccg cgcgcaacat atactcaaac acatatggac ttcattattg aagcctttaa 1320

acatgtgaaa gagaacgcgg cgaatattaa aggattaacc tttacgtacg aaccgaaagt 1380

attgcgtcac ttcaccgcaa aacttaaaga agtttaa 1417

Sequence NO: 4
Sequence length: 1743
Sequence type: DNA
Sequence source: Escherichia coli

atgataaaac cgacgttttt acgccgggtg gccattgctg ctctgctctc aggaagttgt 60

tttagcgccg ccgccgcgcc tcctgcgccg cccgtctcgt atggtgtgga ggaagatgtc 120

ttccacccgg tacgcgcgaa acagggaatg gtagcgtctg tggacgccac tgccactcag 180

gtgggggtgg atattctcaa ggagggcggg aatgccgttg atgccgccgt ggcggtgggc 240

tacgcgctgg cggtaacgca tccgcaggca gggaatctgg gcggtggtgg ttttatgtta 300

atccgctcga aaaatggcaa taccacggct atcgatttcc gcgaaatggc acccgccaaa 360

gcgacccgcg atatgttcct cgatgatcag ggcaacccgg acagcaaaaa atcactcact 420

tcgcatctgg cttccggcac accgggtacg gtagcaggtt tctcgctggc gctggataaa 480

tacggcacca tgccgctgaa caaagtcgtg cagcccgcgt ttaaactggc acgcgatggt 540

tttatcgtta acgacgcgct ggctgacgat ctcaaaacct acggtagcga agtgttgccg 600

aatcacgaaa acagtaaagc tatcttctgg aaagagggcg agccgctgaa aaagggcgac 660

acgctggtgc aggcgaacct ggcaaagagc ctggagatga ttgctgaaaa cggcccggac 720

gaattctata aaggcacgat tgcggaacag atcgcccagg agatgcagaa aaacggtggc 780

ttgatcacta aagaagattt agcagcctat aaagcggtcg aacgcactcc gataagcggc 840

gattatcgcg ggtatcaggt ttactccatg ccaccgccat cctccggcgg gatccatatc 900

gtacaaatcc tcaatattct ggaaaacttc gatatgaaga aatacggctt tggcagcgcc 960

gatgcgatgc aaatcatggc agaagcggag aaatacgcct acgccgaccg ctcggaatat 1020

cttggcgacc cggattttgt caaagtaccg tggcaggcgc tgaccaataa agcctatgcc 1080

aaatctattg ccgatcaaat tgatatcaat aaagcgaagc catccagcga aattcgcccc 1140

ggcaagcttg cgccttatga gagtaatcaa actacccatt actcagtggt ggataaagat 1200

ggtaacgcgg tggcggtgac ctatacgctg aacaccacct tcggtacggg cattgtcgcg 1260

ggcgagagcg gtattctgct taataaccag atggatgatt tctccgccaa accgggcgta 1320

ccgaacgttt acgggctggt gggcggtgat gccaacgccg tcgggccgaa caaacgcccg 1380

ctgtcgtcga tgtcgccgac cattgtggtg aaagacggta aaacctggct ggttaccggt 1440

agcccaggcg gtagccggat catcactaca gtgctgcaaa tggtggtgaa tagcatcgat 1500

tatggcttga acgtcgccga agcgaccaat gcgccgcgtt tccaccatca gtggttgccg 1560

gacgagctgc gtgtcgaaaa agggtttagc ccggatacgc tcaagctgct ggaagcaaaa 1620

ggtcagaaag tggcgctgaa agaggcgatg ggcagtacac aaagcattat ggttgggccg 1680

gacggtgagt tgtacggcgc atccgacccg cgctcggtgg atgatttaac ggcggggtac 1740

taa 1743

Sequence NO: 5
Sequence length: 756
Sequence type: DNA
Sequence source: Escherichia coli

atgaatctta acagtattcc cgcctttgat gacaattaca tctgggtttt gaatgatgaa 60

gcaggtcgct gcctgattgt cgatcccgga gacgcagagc cagtattaaa cgccattgcc 120

gccaataact ggcaaccgga ggccatattt ctcacccacc atcaccacga tcacgttggc 180

ggcgtaaaag aactggtgga aaagtttcca caaattgtgg tgtatggtcc acaagagaca 240

caagataagg gaacaacaca ggtagtcaaa gatggcgaaa ctgccttcgt tttggggcat 300

gaatttagtg taattgctac gccgggtcac actttaggac atatctgtta cttcagtaaa 360

ccttatctat tttgcggcga cacactgttt tctggtgggt gtggtcggtt gtttgaaggg 420

acagcatcac aaatgtatca atcacttaaa aagttaagtg cgttacctga cgatacattg 480

gtatgttgtg ctcatgaata taccttatca aatatgaagt ttgctttgag tattcttccg 540

cacgatttgt ccataaatga ttattatcgt aaagttaagg agttacgggc aaaaaatcaa 600

ataacactac ccgtaattct gaaaaatgag cggcaaatta atgttttttt aagaacggaa 660

gatattgatt taattaatgt aattaatgaa gaaacattat tgcaacaacc tgaagagcgt 720

tttgcatggt taaggtcaaa gaaagatagg ttctga 756

Sequence NO: 6
Sequence length: 648
Sequence type: DNA
Sequence source: *Escherichia coli*

atgaactatc gtattattcc ggtcaccgca ttctcccaga actgttcatt aatctggtgt 60

gaacaaaccc gtctggccgc actggtcgat cctggcggcg atgcggaaaa aatcaaacag 120

gaagttgatg acagcggcct gacactgatg cagatcctgc tgacgcatgg tcatctggac 180

cacgttggcg cagcggcgga actggcgcaa cattacggcg tgccggtttt cggcccggaa 240

aaagaagatg agttctggct gcaaggcttg cctgcgcaaa gtcgtatgtt tggtctggaa 300

gagtgccagc cgctgacgcc agatcgttgg ctgaacgaag gcgataccat cagcataggg 360

aatgtgactt tacaggtgtt acattgccct gggcatacgc cgggtcatgt cgtgtttttt 420

gatgatcggg caaagctgct gatttctggc gatgttattt tcaaaggcgg agtagggcgc 480

agtgacttcc cgcgtggcga tcataatcaa ctgatttctt caatcaaaga taaattgctg 540

ccactggggg atgacgtgat atttattccg ggtcacggac cattatccac acttggttat 600

gaacgcctgc ataatccctt cctgcaagac gaaatgcccg tctggtaa 648

Sequence NO: 7
Sequence length: 837
Sequence type: DNA

Sequence source: *Escherichia coli*

atggaaatgc tcgaagagca ccgctgtttt gaaggctggc agcaacgctg gcgacacgac 60

tccagtacct taaactgccc gatgacgttc agtatctttc tccctccacc tcgtgatcac 120

actccgccac cagtgctgta ctggctttcc ggattaacct gcaatgacga gaacttcacc 180

accaaggcgg gtgcccagcg ggtagcggcg gaactgggga ttgtactggt gatgccagac 240

accagcccgc gcggcgaaaa ggttgccaac gacgatggct acgatttagg ccagggcgca 300

ggcttttatc ttaatgccac gcaaccgccg tgggcgacgc attaccggat gtatgattat 360

ctgcgcgatg aattaccggc gctggttcag tcgcaattta atgtcagcga ccgctgcgcc 420

attagcggtc actcaatggg tggtcacggt gcgctgatta tggcgctgaa aaatccgggt 480

aaatacacca gcgtttcggc ctttgcgcca attgtgaatc cgtgcagcgt cccgtgggga 540

atcaaagcgt ttagcagcta tttaggtgag gacaaaaatg catggctgga atgggacagt 600

tgcgcactga tgtatgccag taacgcgcag gatgcgatcc cgacgcttat cgatcagggc 660

gataatgatc agtttcttgc cgaccagttg caacctgcgg tactggcaga agccgcgcgc 720

cagaaagcgt ggccgatgac gctgcgtatt cagccgggat atgatcacag ttactacttc 780

atcgcctctt ttatagagga tcacctgcgc ttccatgcgc agtatttact gaagtga 837

## Claims

1. A preparation method for S-lactoylglutathione, wherein glutamic acid, glycine, cysteine, and methylglyoxal are used as starting materials and are converted into S-lactoylglutathione under the catalysis of glutathione synthetase and glyoxalase.

2. The preparation method for S-lactoylglutathione as claimed in claim 1, wherein the glutamic acid, glycine, cysteine, and methylglyoxal are used as substrates, a recombinant microorganism comprising a glutathione synthetase-encoding gene and a glyoxalase-encoding gene is added for fermentation, and the glutathione synthetase and the glyoxalase are produced by overexpression of the recombinant microorganism.

3. The preparation method for S-lactoylglutathione as claimed in claim 2, whereinthe glutathione synthetase-encoding gene is selected from any one or more of gshF, gshA, and gshB, and is preferably gshF; further preferably, the nucleotide sequence of the gshF is set forth in SEQ ID NO: 1;
and/or the glyoxalase-encoding gene comprises gloA; preferably, the nucleotide sequence of the gloA is set forth in SEQ ID NO: 2.

4. The preparation method for S-lactoylglutathione as claimed in claim 2 or 3, wherein the preparation method comprises constructing the recombinant microorganism by a genetic engineering method, and the genetic engineering method comprises plasmid expression or genomic integration.

5. The preparation method for S-lactoylglutathione as claimed in claim 4, wherein the recombinant microorganism is constructed by the plasmid expression method;
preferably, the construction method is as follows: a glutathione synthetase-encoding gene and a glyoxalase-encoding gene are obtained by PCR amplification, the obtained genes are co-ligated to a plasmid vector comprising an IPTG inducible promoter and transformed into a competent cell, and after sequencing, a recombinant vector is obtained; and the recombinant vector is transformed into a recipient microorganism to obtain the recombinant microorganism;
preferably, the plasmid vector is selected from any one or two of pZAlac and pZElac.

6. The preparation method for S-lactoylglutathione as claimed in claim 5, wherein
the recombinant vector is pZE-gshF-gloA,
wherein preferably, a construction method for the pZE-gshF-gloA is as follows: a gshF gene and a gloA gene are obtained by PCR amplification, co-ligated to a pZElac vector comprising an IPTG inducible promoter, and transformed into a competent cell, and after sequencing, the plasmid pZE-gshF_gloA is obtained;
preferably, the gshF gene and gloA gene are obtained by PCR amplification using the genome of *Escherichia coli* MG1655 as a template;
preferably, the competent cell is *Escherichia coli* dh5a.

7. The preparation method for S-lactoylglutathione as claimed in claim 5, wherein the recipient microorganism is selected from one or more of *Escherichia coli, Bacillus, Corynebacterium, Saccharomyces,* or *Streptomyces.*

8. The preparation method for S-lactoylglutathione as claimed in claim 5 or 7, wherein the recipient microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

9. The preparation method for S-lactoylglutathione as claimed in any one of claims 5-8, wherein if the recipient microorganism comprises a gene expressing S-lactoylglutathione hydrolase, the gene expressing S-lactoylglutathione hydrolase on the recipient microorganism is required to be knocked out; the gene expressing S-lactoylglutathione hydrolase is, for example, gloB, gloC, or yeiG; preferably, the nucleotide sequence of the gloB is set forth in SEQ ID NO: 5;
preferably, the nucleotide sequence of the gloC is set forth in SEQ ID NO: 6;
preferably, the nucleotide sequence of the yeiG is set forth in SEQ ID NO: 7;
preferably, the recipient microorganism is *Escherichia coli* MG1655ΔgloB, *Escherichia coli* MG1655ΔgloC, *Escherichia coli* MG1655ΔyeiG, *Escherichia coli* MG1655ΔgloBΔgloC, *Escherichia coli* MG1655ΔgloCΔyeiG, *Escherichia coli* MG1655ΔgloBΔyeiG, or *Escherichia coli* MG1655ΔgloBΔgloCΔyeiG.

10. The preparation method for S-lactoylglutathione as claimed in any one of claims 5-9, wherein if the recipient microorganism comprises a gene expressing cysteine hydrolase or glutathione hydrolase, the gene expressing cysteine hydrolase or glutathione hydrolase on the recipient microorganism is further required to be knocked out;
preferably, the gene expressing cysteine hydrolase is tnaA; further preferably, the nucleotide sequence of the tnaA is set forth in SEQ ID NO: 3;
preferably, the gene expressing glutathione hydrolase is ggt; further preferably, the nucleotide sequence of the ggt is set forth in SEQ ID NO: 4;
preferably, the recipient microorganism is *Escherichia coli* MG1655ΔtnaA, *Escherichia coli* MG1655Δggt, or *Escherichia coli* MG1655ΔtnaAΔggt.

11. The preparation method for S-lactoylglutathione as claimed in any one of claims 5-10, wherein the recipient microorganism is *Escherichia coli* MG1655ΔtnaAΔggtΔgloBΔgloCΔyeiG.

12. The preparation method for S-lactoylglutathione as claimed in claim 11, wherein a method for constructing the *Escherichia coli* MG1655ΔtnaAΔggtΔgloBΔgloCΔyeiG comprises the following steps:
1) knocking out the tnaA, ggt, gloB, gloC, and yeiG genes of a wild-type *Escherichia coli* MG1655 strain separately using a homologous recombination method to give five single-deletion bacteria strains;
2) adding the four single-deletion bacteria strains Δggt, ΔgloB, ΔgloC, and ΔyeiG obtained in Step 1) into a wild-type P1 phage separately and culturing to give phages P1vir ggt, P1vir gloB, P1vir gloC, and P1vir yeiG comprising *Escherichia coli* gene fragments with ggt, gloB, gloC, and yeiG knockout characters, respectively; and
3) transfecting the ΔtnaA single-deletion bacteria strain obtained in Step 1), as a recipient strain, by adding the phages obtained in Step 2) sequentially to give *Escherichia coli* MG1655ΔtnaAΔggtΔgloBΔgloCΔyeiG.

13. The preparation method for S-lactoylglutathione as claimed in any one of claims 2-12, wherein during the fermentation, a fermentation temperature is 20-90 °C.

14. The preparation method for S-lactoylglutathione as claimed in any one of claims 1-13, wherein the molar concentration ratio of the glutamic acid to the glycine to the cysteine to the methylglyoxal is 8-12:8-12:6-10:1-4.

15. The preparation method for S-lactoylglutathione as claimed in claim 1, wherein during fermentation, the glutamic acid, glycine, cysteine, and a recombinant microorganism are first added, the fermentation culture is preferably performed for 1-4 h to accumulate glutathione, the methylglyoxal is then added, and the fermentation is continued; preferably, during the fermentation, the concentration of the methylglyoxal in a fermentor is maintained to be 0.2-4 mM by using a slow fed-batch addition method.

16. A preparation method for S-lactoylglutathione, wherein glutathione and methylglyoxal are used as starting materials and are converted into S-lactoylglutathione under the catalysis of a recombinant microorganism overexpressing glyoxalase or its crude enzyme extracts thereof; preferably, a gene expressing S-lactoylglutathione hydrolase in the recombinant microorganism is knocked out, and the gene expressing S-lactoylglutathione hydrolase is, for example, gloB, gloC, or yeiG;
preferably, the nucleotide sequence of the gloB is set forth in SEQ ID NO: 5;
preferably, the nucleotide sequence of the gloC is set forth in SEQ ID NO: 6;
preferably, the nucleotide sequence of the yeiG is set forth in SEQ ID NO: 7;
preferably, the recombinant microorganism is *Escherichia coli* MG1655ΔgloB, *Escherichia coli* MG1655ΔgloC, *Escherichia coli* MG1655ΔyeiG, *Escherichia coli* MG1655ΔgloBΔgloC, *Escherichia coli* MG1655ΔgloCΔyeiG, *Escherichia coli* MG1655ΔgloBΔyeiG, or *Escherichia coli* MG1655ΔgloBΔgloCΔyeiG.

17. A recombinant microorganism for preparing S-lactoylglutathione, wherein the recombinant microorganism overexpresses an endogenous or exogenous glutathione synthetase-encoding gene and glyoxalase-encoding gene;
preferably, the glutathione synthetase-encoding gene is selected from any one or more of gshF, gshA, and gshB, and is preferably gshF; further preferably, the nucleotide sequence of the gshF is set forth in SEQ ID NO: 1;
preferably, the glyoxalase-encoding gene comprises gloA; preferably, the nucleotide sequence of the gloA is set forth in SEQ ID NO: 2;
preferably, the recipient microorganism is selected from one or more of *Escherichia coli, Bacillus, Corynebacterium, Saccharomyces,* or *Streptomyces*.;further preferably, the recipient microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

18. A recombinant DNA or biomaterial for preparing S-lactoylglutathione, wherein the recombinant DNA or biomaterial comprises a glutathione synthetase-encoding gene and a glyoxalase-encoding gene;
preferably, the glutathione synthetase-encoding gene is selected from any one or more of gshF, gshA, and gshB, and is preferably gshF; further preferably, the nucleotide sequence of the gshF is set forth in SEQ ID NO: 1;
preferably, the glyoxalase-encoding gene comprises gloA; preferably, the nucleotide sequence of the gloA is set forth in SEQ ID NO: 2;
preferably, the biomaterial is an expression cassette, a transposon, a plasmid vector, a phage vector, or a virus vector.

19. Use of the recombinant microorganism as claimed in claim 17, the recombinant DNA or biomaterial as claimed in claim 18 for manufacturing S-lactoylglutathione.
